# EUROPEAN PATENT SPECIFICATION

(11) **EP 0 654 473 B1**
(45) Date of publication and mention of the grant of the patent: **08.09.1999**
(21) Application number: 94308492.1
(22) Date of filing: 17.11.1994
(51) Int. Cl.: C07D 473/00, C07D 473/18, A61K 31/52

(54) **Cyclopropane derivatives and antiviral agents containing the same**
Cyclopropan-Derivate und diese enthaltende antivirale Mittel
Dérivés de cyclopropane et agents antiviraux les contenant

(30) Priority: 18.11.1993 JP 28902093
(43) Date of publication of application: 24.05.1995
(73) Proprietor: Ajinomoto Co., Inc., Tokyo 104 (JP)
(72) Inventor: Sekiyama, Takaaki, c/o Central Res. Lab., Kawasaki-shi, Kanagawa-ken (JP); Ikemura, Osamu, c/o Central Res. Lab., Kawasaki-shi, Kanagawa-ken (JP); Onishi, Tomoyuki, c/o Central Res. Lab., Kawasaki-shi, Kanagawa-ken (JP); Tsuji, Takashi, c/o Central Res. Lab., Kawasaki-shi, Kanagawa-ken (JP); Iwayama, Satoshi, c/o Central Res. Lab., Kawasaki-shi, Kanagawa-ken (JP); Suzuki, Katsuya, c/o Central Res. Lab., Kawasaki-shi, Kanagawa-ken (JP); Ohmura, Yuko, c/o Central Res. Lab., Kawasaki-shi, Kanagawa-ken (JP); Okunishi, Masahiko, c/o Central Res. Lab., Kawasaki-shi, Kanagawa-ken (JP)
(74) Representative: Nicholls, Kathryn Margaret

(56) References cited:
- EP-A- 0 099 493
- EP-A- 0 308 065
- EP-A- 0 375 329
- EP-A- 0 502 690
- PATENT ABSTRACTS OF JAPAN vol. 18, no. 337 (C-1217) 27 June 1994 & JP-A-06 080 670 (AJINOMOTO CO INC) 22 March 1994

## Description

The present invention relates to cyclopropane derivatives having an antiviral activity which are useful as an agent for treating various viral diseases, and also relates to an antiviral agent containing the same.

Many nucleic acid derivatives have antiviral activity, and acyclovir, ganciclovir, azidothymidine, etc. have been practically used as an antiviral agent. However, these nucleic acid derivatives have problems since they generally have poor solubility in water. For example, in order to use as an injection, these compounds should be dissolved in a large amount of water and administered by the intravenous drip. Moreover, these compounds can hardly be used as an ophthalmic solution.

Japanese patent application JP-A-59/10587 discloses amino acid esters of acyclovir, particularly, glycine or alanine esters of acyclovir, as a compound having an improved solubility in water as compared with acyclovir. Unexamined published Japanese patent application No. 64-68373 discloses that valine and isoleucine esters of acyclovir have better bioavailability (oral absorbability) after oral administration in comparison with the glycine and alanine esters.

Japanese patent application JP-A-2/218667 discloses amino acid esters of ganciclovir, but does not include oral absorbability data. Accordingly, it is not clear what type of amino acid esters could improve oral absorbability.

On the other hand, Japanese patent application JP-A-5/78357 discloses that the compound represented by the following general formula (II) has potent antiviral activity. 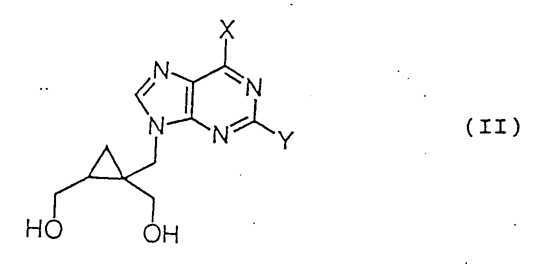 wherein X represents a hydrogen atom, a hydroxyl group, an amino group, or a halogen atom; and Y represents a hydrogen atom, a hydroxyl group, or an amino group.

However, similar to the known nucleic acid derivatives, this compound also has low solubility in water and has problems such as poor oral absorbability and poor transdermal permeability.

The present inventors have conducted extensive studies on the compound represented by the general formula (II) above to improve solubility in water and oral absorbability and, as a result, found that the oral absorbability may be improved and the treatment effect enhanced by esterifying the hydroxyl group of this compound with certain amino acids.

Thus, the present invention relates to a cyclopropane derivative represented by the general formula (I): 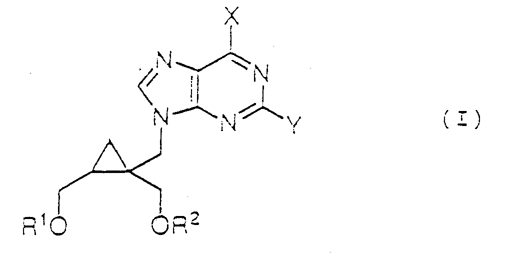 wherein R¹ and R², both represent a group represented by the formula (1): 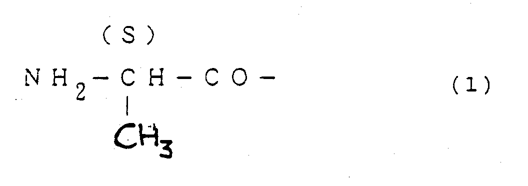 in which X represents a hydrogen atom, a hydroxyl group, an amino group, or a halogen atom; and Y represents a hydrogen atom, a hydroxyl group, or an amino group, or a pharmaceutically acceptable salt thereof, as well as an antiviral agent comprising the same.

The configuration of the compound of the general formula (1) due to the asymmetric carbon atom is S-configuration.

Examples of the halogen atom include fluorine, chlorine, bromine and iodine.

Examples of the pharmaceutically acceptable salt include salts with inorganic acids such as hydrochloric acid, sulfuric acid, nitric acid, phosphoric acid and with organic acid such as acetic acid, lactic acid, citric acid, tartaric acid, maleic acid, fumaric acid, malic acid, succinic acid, and methanesulfonic acid.

Preferable cyclopropane derivatives according to the present invention are shown below.
9-[1'α,2'α-Bis(O-L-alanyloxymethyl)cyclopropan-1'β-yl]methylguanine
9-[1'α,2'α-Bis(O-L-alanyloxymethyl)cyclopropan-1'β-yl]methyl-2-aminopurine
9-[1'α,2'α-Bis(O-L-alanyloxymethyl)cyclopropan-1'β-yl]methyl-2-amino-6-chloropurine

Regarding the stereochemistry of the cyclopropane ring, the cyclopropane derivative of the present invention may be a single stereoisomer, or may be a mixture of stereoisomers, for instance a racemic mixture of optically active isomers. By considering the cyclopropane ring as a plane, the relative configuration of the each compound is represented by the symbols α and β, where α means the group at the lower side position of the cyclopropane plane and β means the group at the upper side position of cyclopropane plane.

The compound of the present invention wherein R¹ and R² are both groups of formula (1) can be prepared by, for example, the following process. 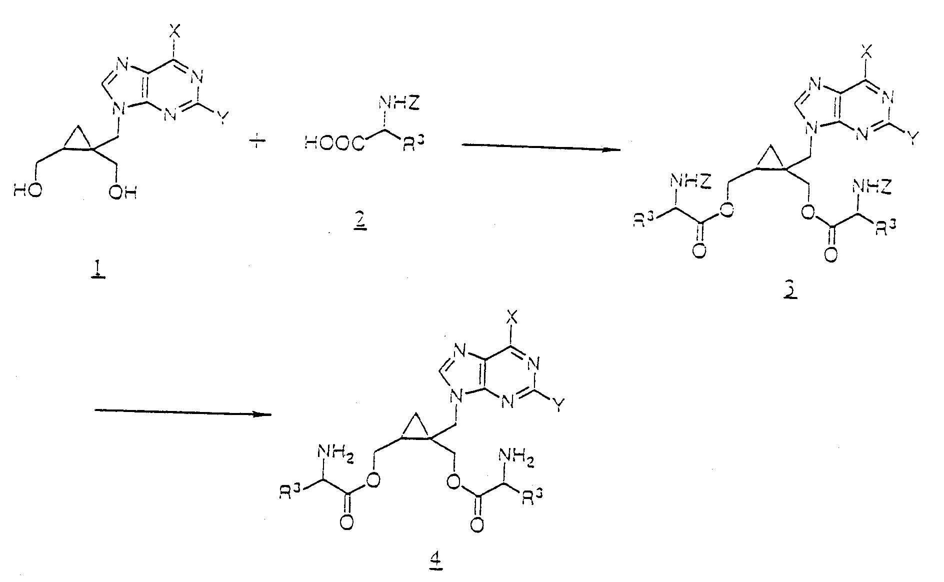 wherein X, and Y, are as defined above, R³ is methyl and Z represents a protective group for an amino group.

First, a cyclopropane derivative (the formula (1)) prepared by the method as described in EP-A-502690
(JP-A-5/78357), etc. and an N-protected amino acid (the formula (2)) are subjected to coupling in a polar solvent such as dimethylformamide, pyridine, acetonitrile, and tetrahydrofuran, using a coupling agent such as carbodiimides (e.g., N,N'-dicyclohexylcarbodiimide) and 2-chloro-1-methylpyridinium tosylate to obtain an ester of the formula (3). The amino-protective group in the ester obtained was removed by an appropriate way to obtain the object compound (the formula (4)).

As the amino-protective group in the formula (2), those generally used for the peptide synthesis can be used for the present invention and examples thereof include a t-butoxycarbonyl group, a benzyloxycarbonyl group, etc. When a t-butoxycarbonyl group is used, the protective group can be removed by making the condition acidic using trifluoroacetic acid, hydrochloric acid, etc. When a benzyloxycarbonyl group is used, the protective group can be removed by catalytic hydrogenation in the presence of palladium-carbon. In addition, an azido group can also be used as a protective group, which can be converted to an amino group by reduction.

Without the coupling agent described above, the reaction can be carried out by using active esters of the N-protected amino acid with N-hydroxysuccinimide, 2,4-dinitrophenol, etc. or acid anhydrides of the N-protected amino acid can be used.

The compound of the present invention has antiviral activity and expected to be useful as an antiviral agent. Examples of the virus include herpes simplex virus, cytomegalovirus, varicella-zoster virus, Epstein-Barr virus, and hepatitis viruses.

The compound of the present invention is used as an antiviral agent by intravenous administration, oral administration, transdermal administration, or ophthalmic administration. Although clinical dose may vary depending on the symptoms and age of the patient, route of administration, and the like, the dose is generally from 0.1 to 500 mg/kg/day. The compound of the present invention is administered as an antiviral composition prepared by mixing the compound with an appropriate pharmaceutical carrier.

Examples of the dosage form of the preparation include injections, tablets, granules, fine granules, powders, capsules, creams, suppositories. Examples of the pharmaceutical carrier include lactose, glucose, D-mannitol, starch, crystalline cellulose, calcium carbonate, kaolin, gelatin, hydroxypropylcellulose, hydroxypropylmethylcellulose, polyvinylpyrrolidone, ethanol, carboxymethylcellulose, carboxymethylcellulose calcium, magnesium stearate, talc, acetylated cellulose, sucrose, titanium oxide, benzoic acid, parahydroxybenzoates, sodium dehydroacetate, acacia, tragacanth, methylcellulose, egg yolk, surfactants, simple syrup, citric acid, distilled water, glycerin, propylene glycol, macrogols, sodium monohydrogenphosphate, sodium dihydrogenphosphate, sodium phosphate, sodium chloride, phenol, Salomethyl, and sodium hydrogensulfite. These pharmaceutical carriers are appropriately mixed with the compound of the present invention depending on the dosage form of the preparation.

The content of the active ingredient according to the present invention in the preparation may vary depending on the dosage form of the preparation and should not particularly be limited, but the active ingredient is generally used in an amount of 0.01 to 80% by weight, preferably 1 to 70% by weight, based on the total amount of the preparation.

### Examples

### Example 1

### Production of 9-[1'S,2'R-bis(O-L-alanyloxymethyl)cyclopropan-1'-yl)methylguanine trihydrochloride

### Process 1

### Production of 9-[1'S,2'R-bis[O-(N-t-butoxycarbonyl)-L-alanyloxymethyl]cyclopropan-1'-yl]methylguanine

In 3 ml of N,N-dimethylformamide was dissolved at 60°C 99.2 mg (0.374 mmol) of 9-[1'S,2'R-bis(hydroxymethyl)cyclopropan-1'-yl]methylguanine obtained by the method as in unexamined published Japanese patent application No. 5-78357, and 195 mg (1.03 mmol) of N-t-butoxycarbonyl-L-alanine, 259 mg (1.26 mmol) of N,N'-dicyclohexylcarbodiimide and 13 mg (0.106 mmol) of 4-dimethylaminopyridine were added to the resulting solution. After stirring the reaction mixture at room temperature for 80 hours, the temperature of the mixture was raised to 60°C, and 195 mg (1.03 mmol) of N-t-butoxycarbonyl-L-alanine and 259 mg (1.26 mmol) of N,N'-dicyclohexylcarbodiimide were added again. The reaction mixture was cooled to room temperature and further stirred for 90 hours. Then, insoluble matters were removed by filtration and the solvent was distilled off under reduced pressure. The residue obtained was subjected to silica gel column chromatography (dichloromethane:methanol=9:1) to give 162.6 mg (0.267 mmol, 70%) of the titled compound.
White solid.
¹H-NMR (CD₃OD) δ: 0.85 (t, J=5.4Hz, 1H), 1.22-1.32 (m, 1H), 1.27 (d, J=7.5Hz, 3H), 1.37 (d, J=7.2Hz, 3H), 1.47 (s, 9H), 1.48 (s, 9H), 1.78-1.90 (m, 1H), 3.97 (d, J=14.4Hz, 1H), 4.04-4.28 (m, 6H), 4.36 (dd, J=6.5, 11.9Hz, 1H), 7.84 (s, 1H) Mass Spectrum (FAB): 607 (MH⁺)

### Process 2

### Production of 9-[1'S,2'R-bis(O-L-alanyloxymethyl)cyclopropan-1'-yl]methylguanine trihydrochloride

In 2 ml of 1.4-dioxane was dissolved 144.8 mg (0.238 mmol) of 9-[1'S,2'R-bis[O-(N-t-butoxycarbonyl)-L-alanyloxymethyl]cyclopropan-1'-yl]methylguanine, and 2 ml of 4N hydrochloric acid-dioxane was added to the resulting solution. After stirring the reaction mixture for 1 hour, the white solid precipitated out was collected by filtration and dried under reduced pressure to quantitatively give 114.3 mg (0.238 mmol) of the titled compound.
White solid
¹H-NMR(DMSO-d6)δ: 0.88 (t, J=5.6Hz, 1H), 1.29 (dd, J=5.6, 9.2Hz, 1H), 1.34 (d, J=6.9Hz, 3H), 1.39 (d, J=7.2Hz, 3H), 1.73-1.84 (m, 1H), 3.95-4.27 (m, 8H), 7.14 (brs, 2H), 8.60 (brs, 3H), 8.68 (brs, 3H), 9.00 (s, 1H), 11.63 (brs, 1H)
High Resolution Mass Spectrum (C₁₇H₂₆N₇O₅, MH⁺):
Calcd., 408.1995; Found, 408.1993

### Comparative Examples

The following compounds were prepared by the similar procedure as in Example 1.

### Comparative Example 1

### Production of 9-[1'S,2'R-bis(O- glycyloxymethyl)cyclopropan-1'-yl]methylguanine trihydrochloride

White solid
¹H-NMR(DMSO-d6)δ: 0.83 (t, J=5.4Hz, 1H), 1.34 (dd, J=5.1, 8.7Hz, 1H), 1.64-1.76 (m, 1H), 3.72-3.86 (m, 4H), 3.91-4.33 (m, 6H), 7.13 (brs, 2H), 8.50 (brs, 3H), 8.57 (brs, 3H), 8.89 (s, 1H), 11.58 (brs, 1H)
High Resolution Mass Spectrum (C₁₅H₂₂N₇O₅, MH⁺):
Calcd., 380.1680; Found, 380.1682

### Comparative Example 2

### Production of 9-[1'S,2'R-bis(O-L-prolyloxymethyl)cyclopropan-1'-yl]methylguanine trihydrochloride

White solid
¹H-NMR(DMSO-d6) δ: 0.89 (t, J=5.7Hz, 1H), 1.28 (dd, J=5.1Hz, 9.0Hz, 1H), 1.74-2.00 (m, 9H), 2.14-2.30 (m, 4H), 4.00-4.40 (m, 8H), 7.04 (brs, 2H), 8.83 (s, 1H), 9.08-9.30 (m, 3H), 10.26-10.50 (m, 3H), 11.53 (brs, 1H)
High Resolution Mass Spectrum (C₂₁H₃₀N₇O₅, MH⁺):
Calcd., 460.2308; Found, 460.2290

### Comparative Example 3

### Production of 9-[1'S,2'R-bis(O-L-phenylalanyloxymethyl)cyclopropan-1'-yl]methylguanine trihydrochloride

White solid
¹H-NMR(DMSO-d6) δ: 0.65 (t, J=5.7Hz, 1H), 1.14 (dd, J=5.7Hz, 9.0Hz, 1H), 1.49-1.61 (m, 1H), 3.00 3.24, (m, 4H), 3.85-4.32 (m, 8H), 6.98 (brs, 2H), 7.16-7.35 (m, 10H), 8.72 (brs, 3H), 8.87 (s, 1H), 8.82 (brs, 3H), 11.49 (brs, 1H)
High Resolution Mass Spectrum (C₂₉H₃₄N₇O₅, MH⁺):
Calcd., 560.2621; Found, 560.2605

### Comparative Example 4

### Production of 9-[1'S,2'R-bis(O-α-L-aspartyl)cyclopropan-1'-yl]methylguanine trihydrochloride

White solid
¹H-NMR(DMSO-d6)δ: 0.76-0.83 (m, 1H), 1.20-1.28 (m, 1H), 1.58-1.68 (m, 1H), 2.90 (d, J=5.1Hz, 2H), 3.66 (d, J=9.6Hz, 2H), 3.92-4.40 (m, 6H), 6.84 (brs, 2H), 8.50-8.80 (m, 7H), 11.34 (brs, 1H)
High Resolution Mass Spectrum (C₁₉H₂₆N₇O₉, MH⁺):
Calcd., 496.1792; Found, 496.1793

### Comparative Example 5

### Production of 9-[1'S,2'R-bis(O-β-L- aspartyl)cyclopropan-1'-yl]methylguanine trihydrochloride

White solid
¹H-NMR(DMSO-d6)δ: 0.75-0.82 (m, 1H), 1.21-1.30 (m, 1H), 1.58-1.68 (m, 1H), 2.80-3.02 (m, 4H), 3.73-3.76 (m, 2H), 3.92-4.38 (m, 6H), 6.85 (brs, 2H), 8.42-8.82 (m, 7H), 11.34 (brs, 1H)
High Resolution Mass Spectrum (C₁₉H₂₆N₇O₉, MH⁺):
Calcd., 496.1792; Found, 496.1790

### Comparative Example 6

### Production of 9-[1'S,2'R-bis(O-β-alanyloxymethyl)cyclopropan-1'-yl]methylguanine trihydrochloride

White solid
¹H-NMR(DMSO-d6)δ: 0.83-0.90 (m, 1H), 1.26-1.44 (m, 5H), 1.65-1.80 (m, 1H), 2.68-2.80 (m, 2H), 2.95-3.03 (m, 2H), 3.82-4.44 (m, 6H), 7.30 (bs, 2H), 8.31 (bs, 3H), 8.73 (bs,3H), 9.06 (d, J=7.8Hz, 1H), 11.78 (bs, 1H)
High Resolution Mass Spectrum (C₁₇H₂₆N₇O₅, MH⁺):
Calcd., 408.1995; Found, 408.2010

### Comparative Example 7

### Production of 9-[1'S,2'R-bis(O-D-alanyloxymethyl)cyclopropan-1'-yl]methylguanine trihydrochloride

White solid
¹H-NMR(DMSO-d6) δ: 0.86 (t, J=5.7Hz, 1H), 1.29 (dd, J=5.7Hz, 9.0Hz, 1H), 1.35 (d, J=7.2Hz, 3H), 1.41 (d, J=6.9Hz, 3H), 1.67-1.78 (m, 1H), 3.90-4.38 (m, 8H), 6.94 (brs, 2H), 8.57 (brs, 3H), 8.64 (brs, 3H), 8.70 (s, 1H), 11.18 (brs, 1H)
High Resolution Mass Spectrum (C₁₇H₂₆N₇O₅, MH⁺):
Calcd., 408.1995; Found, 408.1997

### Comparative Example 8

### Production of 9-[1'S,2'R-bis(O-D-valyloxymethyl)cyclopropan-1'-yl]methylguanine trihydrochloride

White solid
¹H-NMR(DMSO-d6)δ: 0.85 (t, J=5.6Hz, 1H), 0.93 (d, J=6.9Hz, 3H), 0.95 (d, J=6.9Hz, 3H), 0.96 (d, J=7.2Hz, 3H), 0.97 (d, J=6.6Hz, 3H), 1.30 (dd, J=5.6, 8.4Hz, 1H), 1.71-1.81 (m, 1H), 2.06-2.22 (m, 2H), 3.99-4.34 (m, 8H), 6.94 (bs, 2H), 8.52 (bs, 2H), 8.69 (bs, 2H), 11.36 (bs,1H)
High Resolution Mass Spectrum (C₂₁H₃₄N₇O₅, MH⁺):
Calcd., 464.2622; Found, 464.2642

### Comparative Example 9

### Production of 9-[1'S,2'R-bis(O-L-lysyloxymethyl)cyclopropan-1'-yl]methylguanine pentahydrochloride

### Process 1

### Production of 9-[1'S,2'R-bis[O-(α-N-t-butoxycarbonyl)-ω-N-benzyloxycarbonyl-L-lysyloxymethyl]cyclopropan-1'-yl]methylguanine

In 20 ml of N,N-dimethylformamide was dissolved 200 mg (0.74 mmol) of 9-[1'S,2'R-bis(hydroxymethyl)cyclopropan-1'-yl]methylguanine, and 769 mg (2.02 mmol) of α-N-t-butoxycarbonyl-ω-N-benzyloxycarbonyl-L-lysine, 507 mg (2.46 mmol) of N,N'-dicyclohexylcarbodiimide and 26 mg (0.21 mmol) of 4-dimethylaminopyridine were added to the resulting solution. After stirring the resulting mixture at room temperature for 28 hours, 769 mg (2.02 mmol) of α-N-t-butoxycarbonyl-ω-N-benzyloxycarbonyl-L-lysine, 507 mg (2.46 mmol) of N,N'-dicyclohexylcarbodiimide and 26 mg (0.21 mmol) of 4-dimethylaminopyridine were further added. The reaction mixture was stirred at room temperature for 110 hours, insoluble matters were removed by filtration, and the solvent was distilled off under reduced pressure. The residue obtained was subjected to silica gel column chromatography (dichloromethane:methanol=100:5) to give 440 mg (0.444 mmol, 58.9%) of the titled compound.
White solid
¹H-NMR(CDCl₃)δ: 0.68-0.72 (m, 1H), 1.02-1.15 (m, 1H), 1.38 (s, 9H), 1.42 (s, 9H), 1.22-1.80 (m, 13H), 3.10-3.20 (m, 4H), 3.70-4.35 (m, 8H), 5.07 (s, 4H), 5.24-5.32 (m, 1H), 5.36-5.44 (m, 1H), 7.26-7.34 (m, 10H), 7.63 (s, 1H)
Mass Spectrum (FAB): 990 (MH⁺)

### Process 2

### Production of 9-[1'S,2'R-bis(O-L-lysyloxymethyl)cyclopropan-1'-yl]methylguanine pentahydrochloride

In 5 ml of methanol was dissolved 365 mg (0.369 mmol) of 9-[1'S,2'R-bis[O-(α-N-t-butoxycarbonyl)-ω-benzyloxycarbonyl-L-lysyloxymethyl]cyclopropan-1'-yl]methylguanine, and 150 mg of 5% palladium-carbon (52.4% wet) was added to the resulting solution. The reaction mixture was stirred at room temperature for 3 hours under hydrogen atmosphere and filtered. The solvent was distilled off from the filtrate, the residue was dissolved in 4 ml of methanol, and 4 ml of 4N hydrochloric acid-dioxane was added thereto. The mixture was stirred at room temperature for 6 hours, and the solvent was distilled off under reduced pressure. The residue obtained was reprecipitated from methanol-ethyl acetate, and the precipitate was collected by filtration and dried under reduced pressure to give 241.7 mg (0.343 mmol, 93.3%) of the titled compound.
White solid
¹H-NMR(DMSO-d6) δ: 0.92 (t, J=5.7Hz, 1H), 1.29 (dd, J=5.7, 9.0Hz, 1H), 1.32-1.83 (m, 13H), 2.70-2.83 (m, 4H), 3.85-4.30 (m, 8H), 7.04 (brs, 2H), 8.12 (brs, 6H), 8.62-8.84 (m, 7H), 11.52 (brs, 1H)
High Resolution Mass Spectrum (C₂₃H₄₀N₉O₅, MH⁺):
Calcd., 522.3152; Found, 522.3178

### Comparative Example 10

### Production of 9-[1'S,2'R-bis(O-L-seryloxymethyl)cyclopropan-1'-yl]methylguanine trihydrochloride

### Process 1

### Production of 9-[1'S,2'R-bis[O-(N-t-butoxycarbonyl)-O-benzylseryloxymethyl]cyclopropan-1'-yl]methylguanine

In 20 ml of N,N-dimethylformamide was dissolved 200 mg (0.754 mmol) of 9-[1'S,2'R-bis(hydroxymethyl)cyclopropan-1'-yl]methylguanine, and 597 mg (2.02 mmol) of N-t-butoxycarbonyl-O-benzyl-L-serine, 507 mg (2.46 mmol) of N,N'-dicyclohexylcarbodiimide and 26 mg (0.21 mmol) of 4-dimethylaminopyridine were added to the resulting solution. After stirring the reaction mixture at room temperature for 28 hours, 597 mg (2.02 mmol) of N-t-butoxycarbonyl-O-benzyl-L-serine, 507 mg (2.46 mmol) of N,N'-dicyclohexylcarbodiimide and 26 mg (0.21 mmol) of 4-dimethylaminopyridine were further added. The reaction mixture was stirred at room temperature for 110 hours, insoluble matters were removed by filtration, and the solvent was distilled off under reduced pressure. The residue obtained was subjected to silica gel column chromatography (dichloromethane:methanol=100:5) to give 427.7 mg (0.522 mmol, 69.2%) of the titled compound.
White solid
¹H-NMR(CDCl₃)δ: 0.60-0.68 (m, 1H), 1.00-1.10 (m,1 H), 1.39 (s, 9H), 1.41 (s, 1H), 1.52-1.65 (m, 1H), 3.60-3.94 (m, 6H), 4.02-4.30 (m, 4H), 4.36-4.58 (m, 2H), 4.46 (s, 2H), 4.50 (s, 2H), 5.42 (d, J=8.1Hz, 1H), 5.61 (d, J=8.1Hz, 1H), 6.00 (brs, 2H), 7.20-7.32 (m, 10H), 7.59 (s, 1H)
Mass Spectrum (FAB): 820 (MH⁺)

### Process 2

### Production of 9-[1'S,2'R-bis(O-L-seryloxymethyl)cyclopropan-1'-yl]methylguanine trihydrochloride

In 5 ml of methanol was dissolved 300 mg (0.366 mmol) of 9-[1'S,2'R-bis[O-(N-t-butoxycarbonyl)-O-benzyloxyseryloxymethyl]cyclopropan-1'-yl]methylguanine, and 150 mg of 5% palladium-carbon (52.4% wet) was added to the resulting solution. The reaction mixture was stirred at 40°C for 10 hours under hydrogen atmosphere of 3 kgf/cm², and filtered. The solvent was distilled off from the filtrate under reduced pressure and 4 ml of 4N hydrochloric aciddioxane was added to the residue. The resulting mixture was stirred at room temperature for 1 hours and the solvent was distilled off under reduced pressure. The residue obtained was reprecipitated from methanol-diethyl ether, and the precipitate was collected by filtration and dried under reduced pressure to give 180.5 mg (0.329 mmol, 89.9%) of the titled compound.
White solid
¹H-NMR(DMSO-d6) δ: 0.84 (t, J=5.7Hz, 1H), 1.28 (dd, J=5.1, 8.7Hz, 1H), 1.62-1.72 (m, 1H), 3.70-3.92 (m, 6H), 3.94-4.28 (m, 6H), 6.80 (brs, 2H), 8.38 (s, 1H), 8.54 (brs, 3H), 8.60 (brs, 3H), 11.18 (brs, 1H)
High Resolution Mass Spectrum (C₁₇H₂₆N₇O₇, MH⁺):
Calcd., 440.1894; Found, 440.1894

### Example 2

### Production of 9-[1'S,2'R-bis(O-L-alanyloxymethyl)cyclopropan-1'-yl]methylguanine dihydrochloride

### Process 1

### Production of 9-[1'S,2'R-bis[O-(N-benzyloxycarbonyl)-L-alanyloxymethyl]cyclopropan-1'-yl]methylguanine

In 50 ml of N,N-dimethylformamide was dissolved 200 mg (0.754 mmol) of 9-[1'S,2'R-bis(hydroxymethyl)cyclopropan-1'-yl]methylguanine, and 451 mg (2.02 mmol) of N-benzyloxycarbonyl-L-alanine, 507 mg (2.46 mmol) of N,N'-dicyclohexylcarbodiimide and 26 mg (0.21 mmol) of 4-dimethylaminopyridine were added to the resulting solution. After stirring the reaction mixture at room temperature for 48 hours, 451 mg (2.02 mmol) of N-benzyloxycarbonyl-L-alanine, 507 mg (2.46 mmol) of N,N'-dicyclohexylcarbodiimide and 26 mg (0.21 mmol) of 4-dimethylaminopyridine were further added. The reaction mixture was stirred at room temperature for 96 hours, insoluble matters were removed by filtration, and the solvent was distilled off under reduced pressure. The residue obtained was subjected to silica gel column chromatography (dichloromethane:methanol=100:5) to give 429 mg (0.635 mmol, 84.3%) of the titled compound.
White solid
¹H-NMR(DMSO-d6)δ: 0.72 (m, 1H), 1.13 (m, 1H), 1.17 (d, J=7.5Hz, 3H), 1.26 (d, J=7.5Hz, 3H), 1.59 (m, 1H), 3.8-4.2 (m, 8H), 5.03 (s, 4H), 6.27 (bs, 2H), 7.2-7.4 (m, 10H), 7.63 (s, 1H), 7.68 (d, J=7.5Hz, 1H), 7.75 (d, J=7.5Hz, 1H), 10.51 (bs, 1H)
Mass Spectrum (FAB): 676 (MH⁺)

### Process 2

### Production of 9-[1'S,2'R-bis(O-α-L-alanyloxymethyl)cyclopropan-1'-yl]methylguanine dihydrochloride

In 30.2 ml of methanol was dissolved 2.042 g (3.02 mmol) of 9-[1'S,2'R-bis[O-(N-t-butoxycarbonyl)-α-L-alanyloxymethyl]cyclopropan-1'-yl]methylguanine, and 408 mg of 10% palladium-carbon was added to the resulting solution. The reaction mixture was stirred for 24 hours under hydrogen atmosphere. Insoluble matters were removed by filtration and the solvent was distilled off under reduced pressure to quantitatively give 1.61 g of the titled compound.
White solid
¹H-NMR(DMSO-d6)δ: 0.84 (t, J=5.4Hz, 1H), 1.19 (dd, J=5.4, 8.7Hz, 1H), 1.30 (d, J=7.2Hz, 3H), 1.39 (d, J=7.2Hz, 3H), 1.67-1.75 (m, 1H), 3.90-4.26 (m, 8H), 6.49 (bs, 2H), 7.78 (s, 1H), 8.50 (bs, 6H), 10.72 (bs, 1H)
Mass Spectrum (FAB): 408 (MH⁺)
High Resolution Mass Spectrum (C₁₇H₂₆N₇O₅, MH⁺):
Calcd., 408.1995; Found, 408.1984

### Test Example 1

### Measurement of Oral Absorbability

Each of the test compounds was dissolved in 0.2 ml of physiological saline and orally administered to 5-week-age male ICR mouse in a dose of 0.1 mmol/kg. After 15 minutes, 30 minutes, 1 hour, 2 hours, or 4 hours from the administration, blood was collected from three mice, respectively, and centrifuged at 3,000 rpm for 10 minutes to obtain a plasma. A 20 % trichloroacetic acid aqueous solution (100 µl) was added to the plasma (100 µl) and the resulting mixture was shaken and then centrifuged at 8,000 rpm for 1 minutes to obtain a supernatant. The concentration of 9-[1'S,2'R-bis(hydroxymethyl)cyclopropan-1'-yl]methylguanine in the supernatant was measured by HPLC and the area under the blood concentration time curve (AUC) was calculated. The results are shown in the following Table 1.

**Table 1**

| Test Compound | AUC (µM×hr) |
|---|---|
| Compound of Example 1 | 49 |
| Compound of Comparative Example 1 | 14 |
| Compound of Comparative Example 2 | 12 |
| Compound of Comparative Example 3 | 14 |
| Compound of Comparative Example 4 | 10 |
| Compound of Comparative Example 5 | 0 |
| Compound of Comparative Example 6 | 1 |
| Compound of Comparative Example 7 | 6 |
| Compound of Comparative Example 8 | 22 |
| Compound of Comparative Example 9 | 20 |
| Compound of Comparative Example 10 | 25 |
| 9-[1'S,2'R-bis(hydroxymethyl) cyclopropan-1'-yl]methylguanine | 16 |
| 9-(2-O-L-valyloxyethoxy)methylguanine | 40 |

As clearly shown by the results above, the compound of the present invention has superior oral absorbability to 9-[1'S,2'R-bis(hydroxymethyl)cyclopropan-1'-yl]methylguanine, which is the equivalent or more excellent oral absorbability in comparison with 9-(2-O-L-valyloxyethoxy)methylguanine.

### Test Example 2

### Efficacy Test by Oral Administration

A 4-week-age male CDF-1 mouse was intraperitoneally infected with HSV-1 (Tomioka strain) in an amount of 2.4 × 10⁵ pfu/mouse. Three hours after the infection, administration of each test compound was started. The test compound was dissolved in 0.2 ml of distilled water for injection use and intragastrically administered via catheter once a day for 5 days in a dose of 0.625, 1.25, or 2.5 µmol/kg/day. Each of the test groups consisted of 10 mice, and the survival ratio after 21 days from the administration and the average survival days were obtained. The results are shown in the following Table 2.

**Table 2**

| Test Compound | Dose (µmol/kg) | Survival Ratio after 21 days | Average Survival days |
|---|---|---|---|
| Compound of Example 1 | 2.5 | 100 | |
| | 1.25 | 60 | 14.3±4.1 |
| | 0.625 | 40 | 9.7±2.7 |
| 9-[1'S,2'R-bis(hydroxymethyl)cyclopropan-1'-yl]methylguanine | 2.5 | 60 | 11.3±3.1 |
| | 1.25 | 0 | 9.4±1.9 |
| | 0.625 | 0 | 7.8±1.2 |

As clearly shown by the results above, one of the compounds of the present invention, i.e. 9-[1'S,2'R-bis(O-L-alanyloxymethyl)cyclopropan-1'-yl]methylguanine, has superior efficacy to 9-[1'S,2'R-bis(hydroxymethyl)cyclopropan-1'-yl]methylguanine.

### Example 8

### Production of Preparation for Injection and Intravenous Drip

In 600 ml of the purified water was dissolved 1 g of 9-[1'S,2'R-bis(O-L-alanyloxymethyl)cyclopropan-1'-yl]methylguanine, and resulting solution was sterilized by filtration using a Millipore filter. A 15 g portion of the filtrate was taken and put into a 20 ml vial and lyophilized to give a lyophilized preparation containing 25 mg of the active compound per one vial.

### Example 9

### Production of Tablet

Ten grams of 9-[1'S,2'R-bis(O-L-alanyloxymethyl)cyclopropan-1'-yl]methylguanine, 40 g of lactose, 49 g of crystalline cellulose, and 1 g of magnesium stearate were mixed well, and made into tablets using a tabletting machine to give tablets containing 250 mg of the active compound per one tablet.

### Example 10

### Production of Granule

Ten grams of 9-[1'S,2'R-bis(O-L-alanyloxymethyl)cyclopropan-1'-yl]methylguanine, 90 g of lactose, and 100 g of crystalline cellulose were mixed well, and the mixture was compressed using a roller-type compressor, pulverized, and then sieved to give 20 to 80-mesh granules.

### Example 11

### Production of Ointment

A mixture of 1 g of 9-[1'S,2'R-bis(O-L-alanyloxymethyl)cyclopropan-1'-yl]methylguanine, 6 g of propylene glycol, 4 g of carboxymethylcellulose, and 89 g of white petrolatum was heated to about 60°C and melt-mixed to give an ointment containing 1% by weight of the active compound.

### Example 12

### Production of Ophthalmic Solution

In 100 ml of the pure water were dissolved 1 g of 9-[1'S,2'R-bis(O-L-alanyloxymethyl)cyclopropan-1'-yl]methylguanine and 1 g of sodium chloride, and the pH of the resulting solution was adjusted to 7.5 using sodium monohydrogenphosphate to give an ophthalmic solution containing about 1% by weight of the active compound.

The cyclopropane derivatives of the present invention are excellent in solubility in water and can provide antiviral agents excellent in oral absorbability and treatment effect.

## Claims

1. A cyclopropane derivative represented by the general formula (I): 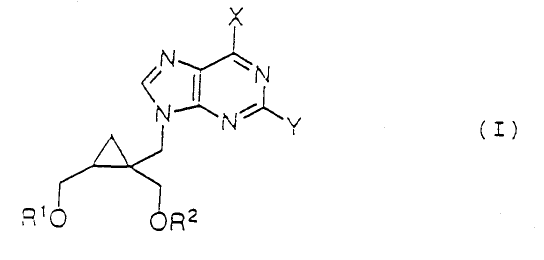 wherein R¹ and R², each represent a group represented by the formula (1): 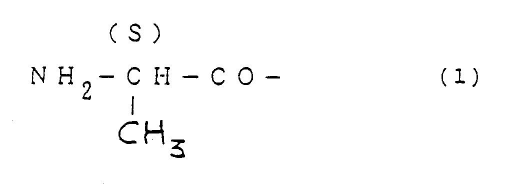 in which X represents a hydrogen atom, a hydroxyl group, an amino group, or a halogen atom; and Y represents a hydrogen atom, a hydroxyl group, or an amino group; or a pharmaceutically acceptable salt thereof.

2. The cyclopropane derivative or claim 1, wherein X is a hydroxy group and Y is an amino group, or a pharmaceutically acceptable salt thereof.

3. 9-[1'S,2'R-Bis(O-L-alanyloxymethyl)cyclopropan-1'β-yl]methylguanine or a pharmaceutically acceptable salt thereof.

4. A pharmaceutical composition comprising a cyclopropane derivative or pharmaceutically acceptable salt thereof of any one of claims 1 to 3 and acceptable excipient, diluent or carrier.

5. A compound of any one of claims 1 to 3 for pharmaceutical use.

6. Use of a compound of any one of claims 1 to 3 in the manufacture of a medicament for antiviral use.

7. A method for the production of a compound of claim 1 comprising deprotection of a compound of formula 3 wherein Z is an amino protecting group, and X, and Y are as defined in claim 1.

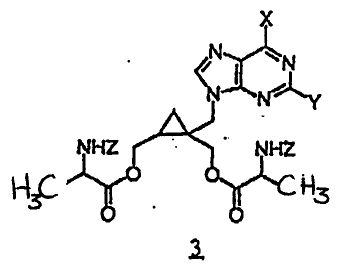

8. A method for the production of a compound of claim 1, comprising esterifying a compound of formula 1 with an acid of formula 2 to form a compound of formula 3 and deprotecting the compound of formula 3 to form a compound of formula 4 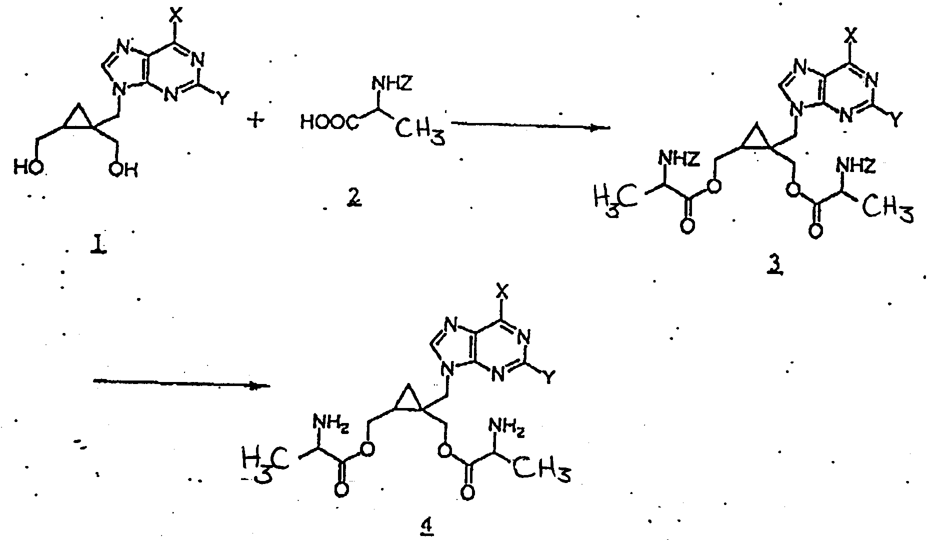 wherein X, and Y, are as defined above, and Z represents a protective group for an amino group.

9. An intermediate of formula 3 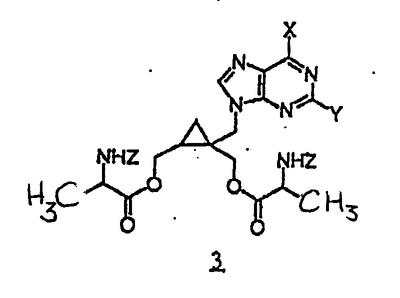 wherein X, and Y are as defined in claim 1 and Z is an amino protecting group.

## Patentansprüche

1. Cyclopropanderivat der allgemeinen Formel (I) 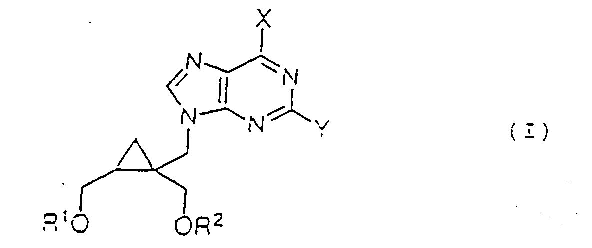 in der R¹ und R² eine Gruppe der Formel (1) bedeuten: 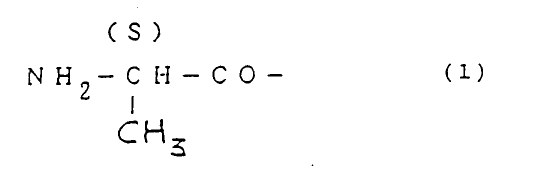 worin X ein Wasserstoffatom, eine Hydroxylgruppe, eine Aminogruppe oder ein Halogenatom bedeutet und Y ein Wasserstoffatom, eine Hydroxylgruppe oder eine Aminogruppe bedeutet,
sowie ein pharmazeutisch verträgliches Salz davon.

2. Cyclopropanderivat nach Anspruch 1, worin X eine Hydroxygruppe bedeutet und Y eine Aminogruppe bedeutet, oder ein pharmazeutisch verträgliches Salz davon.

3. 9-[1'S,2'R-Bis-(O-L-alanyloxymethyl)-cyclopropan-1'-β-yl]-methylguanin oder ein pharmazeutisch verträgliches Salz davon.

4. Pharmazeutische Zusammensetzung, enthaltend ein Cyclopropanderivat oder ein pharmazeutisch verträgliches Salz davon nach einem der Ansprüche 1 bis 3 und ein verträgliches Exzipiens, Verdünnungsmittel oder Trägerstoff.

5. Verbindung nach einem der Ansprüche 1 bis 3 zur pharmazeutischen Verwendung.

6. Verwendung einer Verbindung nach einem der Ansprüche 1 bis 3 bei der Herstellung eines Arzneimittels für antivirale Zwecke.

7. Verfahren zur Herstellung einer Verbindung nach Anspruch 1, umfassend die Schutzgruppenentfernung an einer Verbindung der Formel 3, worin Z eine Aninoschutzgruppe bedeutet und X und Y die in Anspruch 1 definierten Bedeutungen haben:

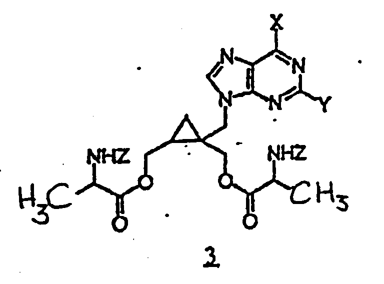

8. Verfahren zur Herstellung einer Verbindung nach Anspruch 1, umfassend die Veresterung einer Verbindung der Formel 1 mit einer Säure der Formel 2 unter Bildung einer Verbindung der Formel 3 und das Entfernen der Schutzgruppe an der Verbindung der Formel 3 unter Bildung einer Verbindung der Formel 4: 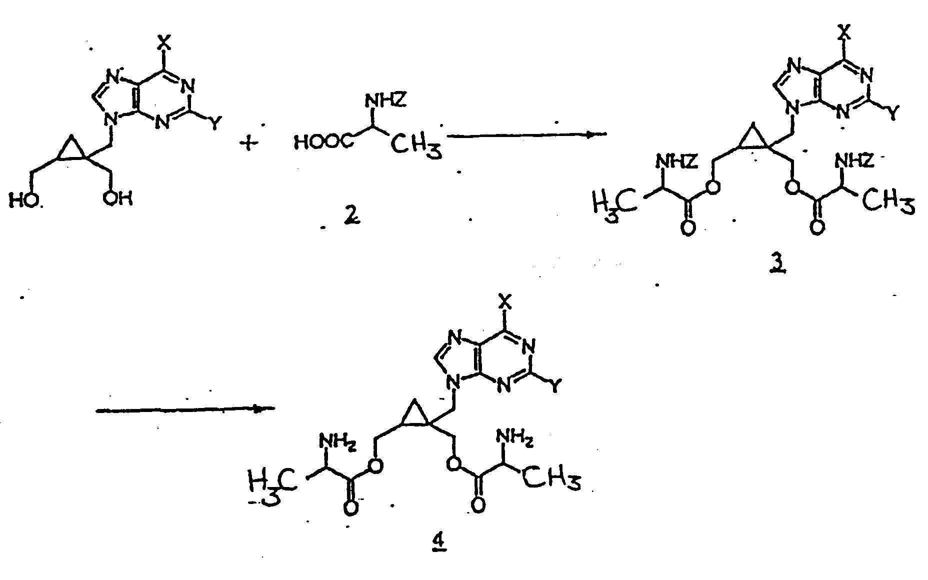 worin X und Y die vorstehend definierten Bedeutungen haben und Z eine Schutzgruppe für eine Aminogruppe bedeutet.

9. Zwischenprodukt der Formel 3 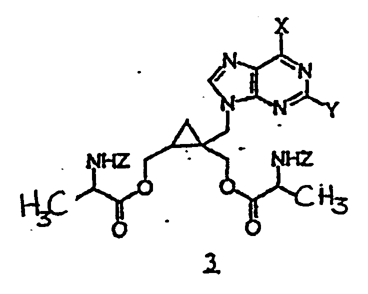 worin X und Y die in Anspruch 1 definierten Bedeutungen haben und Z eine Aminoschutzgruppe bedeutet.

## Revendications

1. Dérivé de cyclopropane représenté par la formule générale (I) 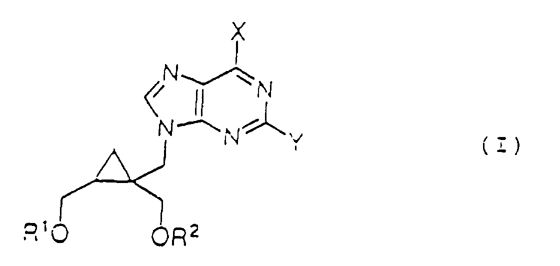 dans laquelle R¹ et R² représentent chacun un groupe représenté par la formule (1): 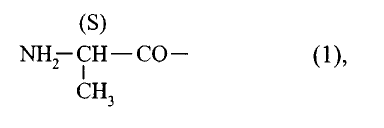 X représente un atome d'hydrogène, un groupe hydroxyle, un groupe amino ou un atome d'halogène; et Y représente un atome d'hydrogène, un groupe hydroxyle ou un groupe amino,
ou un de ses sels pharmaceutiquement acceptables.

2. Dérivé de cyclopropane selon la revendication 1, dans lequel X est un groupe hydroxy et Y est un groupe amino, ou un de ses sels pharmaceutiquement acceptables.

3. La 9-[1'S,2'R-bis(O-L-alanyloxyméthyl)cyclopropane-1'β-yl]méthylguanine ou un de ses sels pharmaceutiquement acceptables.

4. Composition pharmaceutique comprenant un dérivé de cyclopropane selon l'une quelconque des revendications 1 à 3 ou un de ses sels pharmaceutiquement acceptables et un excipient, diluant ou support acceptable.

5. Composé selon l'une quelconque des revendications 1 à 3 pour l'utilisation en pharmacie.

6. Utilisation d'un composé selon l'une quelconque des revendications 1 à 3 dans la fabrication d'un médicament à usage antiviral.

7. Procédé de production d'un composé selon la revendication 1, comprenant l'élimination des groupes protecteurs d'un composé de formule 3 dans laquelle Z est un groupe protecteur d'amino, et X et Y ont la définition indiquée dans la revendication 1.

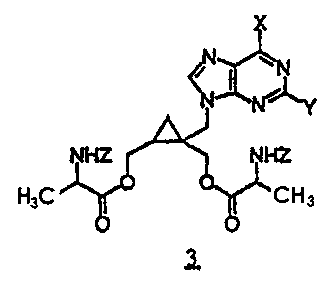

8. Procédé de production d'un composé selon la revendication 1, dans lequel on estérifie un composé de formule 1 avec un acide de formule 2 pour former un composé de formule 3 et on élimine les groupes protecteurs du composé de formule 3 pour former un composé de formule 4 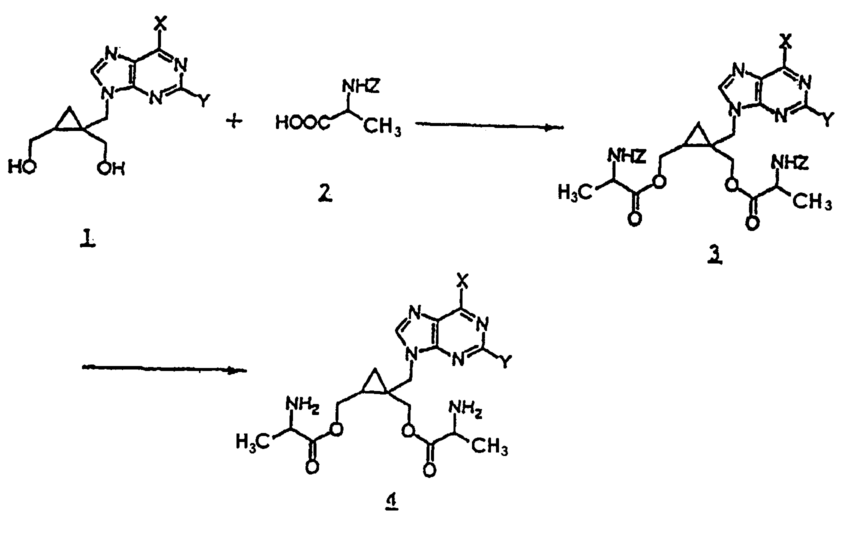 où X et Y ont la définition indiquée ci-dessus, et Z représente un groupe protecteur pour un groupe amino.

9. Intermédiaire de formule 3 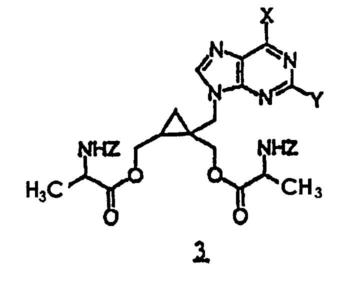 dans laquelle X et Y ont la définition indiquée dans la revendication 1 et Z est un groupe protecteur d'amino.
